Europäisches Patentamt

(19) European Patent Office

Office européen des brevets

(11) Publication number: **0 224 080 B1**

(12) **EUROPEAN PATENT SPECIFICATION**

(45) Date of publication of patent specification: **01.07.92**

(51) Int. Cl.5: **C23C 14/06**, C23C 14/34, A61F 2/30

(21) Application number: **86115254.4**

(22) Date of filing: **04.11.86**

(54) **Improvements in and relating to the manufacture of prosthetic devices.**

(30) Priority: **26.11.85 US 801753**

(43) Date of publication of application:
**03.06.87 Bulletin 87/23**

(45) Publication of the grant of the patent:
**01.07.92 Bulletin 92/27**

(84) Designated Contracting States:
**AT BE CH DE ES FR GB GR IT LI LU NL SE**

(56) References cited:
**EP-A- 0 102 328
DE-A- 3 116 040
US-A- 3 907 660**

**Carbon, 1976, Volume 14, pages 329-331.**

(73) Proprietor: **SORIN BIOMEDICA S.p.A.
Strada per Crescentino
I-13040 Saluggia (Vercelli)(IT)**

(72) Inventor: **Vallana, Franco
Via S. Massimo 28
I-10123 Torino(IT)**
Inventor: **Arru, Pietro
Piazza Arbarello 5
I-10122 Torino(IT)**

(74) Representative: **Bosotti, Luciano et al
c/o Jacobacci-Casetta & Perani S.p.A. Via
Alfieri, 17
I-10121 Torino(IT)**

## Description

The invention relates to improvements in and relating to the manufacture of prosthetic devices having a thin continuous biocompatible film of carbon firmly adherent to a substrate, such as disclosed in EP-A-0 102 328, by the same applicant, or, in a somewhat different context in EP-A-0 029 787 or DE-A-3 116 040.

In EP-A-0 102 328 a method and apparatus is disclosed for manufacturing prosthetic devices such as cardiac valve or vascular prostheses wherein cathode sputtering of the carbon coating is used. Prior to the use of cathode sputtering a satisfactory prosthetic device having a thin continuous film of biocompatible carbon firmly adherent to a substrate had not been generally available. Existing carbon films did not exhibit adherence to their substrate or had not been in a fully biocompatible form since the mechanical properties of the substrates were adversely affected by the methods and apparatus employed to form the films thereon. Representative of such prior techniques for coating a carbon film on a substrate of a prosthetic device include chemical vapor deposition, thermal evaporation by means of an electron beam and ion plating.

The article by Z. Marinkovic and R. Roy, which appeared in Carbon, 1976, Volume 14, pages 329-331, discloses preparation and properties of glassy carbon films sputtered by means of RF sputtering.

In early chemical vapor deposition techniques for forming prosthetic devices, a source for carbon was decomposed at relatively high temperatures and the carbon was deposited in thick layers (e.g., at least 25 microns) on a substrate (see, for example, US-A-3 526 005 US-A-3 685 059 and US-A-3 707 006). Since these techniques typically employed high temperatures e.g., in excess of 1000 °C, it was necessary to select substrate materials which were not adversely affected by the high temperatures employed during the process. As a result, the substrate materials employed for the making of prosthetic devices were limited. Such high temperature techniques cannot take advantage of the desirable properties of available low temperature substrate materials.

Efforts to overcome drawbacks attendant to high temperatures have included initially coating the substrates with high temperature organic polymers and then depositing the carbon thereon (see, for example, US-A-3 952 334). Such techniques are relatively complex and have not met with significant success.

These techniques also have been relatively costly and have produced a layer of carbon which has not satisfactorily adhered to the substrate. In use it has been found that the carbon layer tends to separate from the substrate and allows body fluids and tissue to attack and degrade the prosthetic device.

Attempts to modify existing chemical vaporization techniques shall continue. Recently, it has been suggested that catalysts be used to lower processing temperatures. However, such suggestions add to the complexity and costs of the process and result in films which, in use, have experienced undesirable degradation and separation from the substrates intended to be protected.

Other techniques which have been tried and which have not met with success include electron beam evaporation and ion plating of carbon on a substrate. It has been found that the infrared radiation generated by the electron beam evaporation technique causes degradation of the substrate. In addition, the pressures used are so low that the carbon atoms tend to flow in a very straight line and do not uniformly coat a substrate which normally has a morphologically complex surface.

Further, with the electron beam technique, the deposited carbon is not biocompatible and does not firmly adhere to the substrate. The carbon on the substrate is in form of graphite which has been found not to be biocompatible. Also, the deposited carbon tends to separate and is removed from the substrate during use causing body fluids and tissue to attack and degrade the prosthetic device.

With respect to the ion plating technique, it is a modification of vacuum evaporation in which the substrate is held during the deposition at a high negative potential so that it is bombarded by positive ions, in order to improve the adhesion and the structural properties of the film. Obviously this technique cannot be advantageously employed with electrically insulated substrates, like polymers. In fact, it is difficult to electrically bias non-conductive desirable substrates, such as polyester resins or polytetrafluoroethylene sold under the trademarks DACRON and TEFLON and other relatively low melting polymers.

Consequently, the invention disclosed in EP-A-0 102 328 has given a valuable response to the need for prosthetic devices which have a thin biocompatible carbon coating firmly adherent to the substrate, such as low temperature DACRON and TEFLON, etc., and which are relatively simple and inexpensive to produce.

The present invention, as called for in Claims 1 and 11 and the appended subclaims, relates to some significant, unexpected improvements of the results obtained using the method disclosed in EP-A-0 102 328.

In each embodiment of the invention there is obtained the desired prosthetic device including a biocompatible film of turbostratic carbon of the desired thickness and uniformity, deposited on and firmly adherent to a substrate so that the device has and maintains its integrity.

In the following a detailed description of the invention is provided in connection with accompanying drawings of illustrative embodiments of the invention.

FIGURE 1 is an elevational view of a preferred embodiment of a triode sputtering vacuum deposition apparatus for carrying out the present invention;

FIGURE 2 is another view of the triode sputtering vacuum apparatus of FIGURE 1 wherein the bell jar (ionization and sputtering chambers) is hydraulically raised from its base to expose the target and surrounding anode;

FIGURE 3 is a top view of the apparatus of FIGURE 1, wherein the bell jar has been rotated out of the way for purpose of loading substrates to be coated and for removing coated substrates;

FIGURE 4 is a longitudinal sectional view of the sputtering apparatus of FIGURES 1-3, illustrating the ionization and sputtering chambers, the target and anode, and an internal housing including disks for holding the substrates to be coated;

FIGURE 5 is a perspective view of a lower rotatable ring of the apparatus of FIGURES 1-3, upon which the internal housing is placed, and an upper stationary ring for engaging and causing the disks to rotate about their centers as the housing and disks are rotated by the lower rotatable ring about the longitudinal axis of the bell jar;

FIGURE 6 is a schematic view of the apparatus of FIGURE 1 illustrating the electric circuits and heating, cooling, hydraulic and vacuum systems utilized by the invention, along with showing the plasma beam generated in the ionization chamber centered and directed to the target area;

FIGURE 7 is a perspective view of a disk and frame for holding a substrate which is to be coated in accordance with the present invention;

FIGURE 8 is a perspective view of the coated substrate of the invention after it has been removed from the disk and frame shown in FIGURE 7;

FIGURE 8A is a cross sectional view of the coated substrate of FIGURE 8;

FIGURE 9 is a schematic view of a component for a cardiac valve encased within the coated substrate of FIGURE 8;

FIGURE 10, 11, 11A and 12 are similar to FIGURES 7, 8, 8A and 9, but illustrate the use of a disk and frame (FIGURE 10) for coating a masked substrate which allows the substrate to be coated only over selected portions (FIGURE 11 and 11A) for use in a ring of a mechanical cardiac valve (FIGURE 12);

FIGURE 13 is a perspective view of an apparatus of the invention for coating tubing, e.g., vascular tubing, which can be positioned in the apparatus of FIGURES 1-5;

FIGURE 13A is a cross sectional view of the coated tubing prior to inversion;

FIGURE 13B is a cross sectional view of the coated tubing after inversion;

FIGURE 14 is a perspective view of another apparatus of the invention for coating suture yarn which also can be positioned in the device of FIGURES 1-5;

FIGURE 15 is an enlarged view of the frame and suture yarn of FIGURE 14; and

FIGURE 16 is a cross sectional view of the coated yarn.

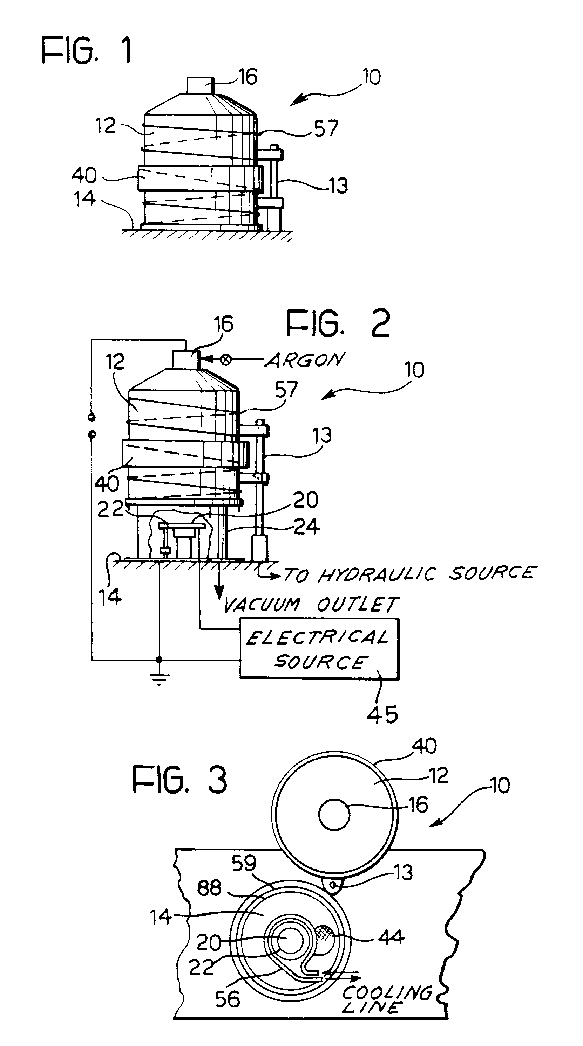

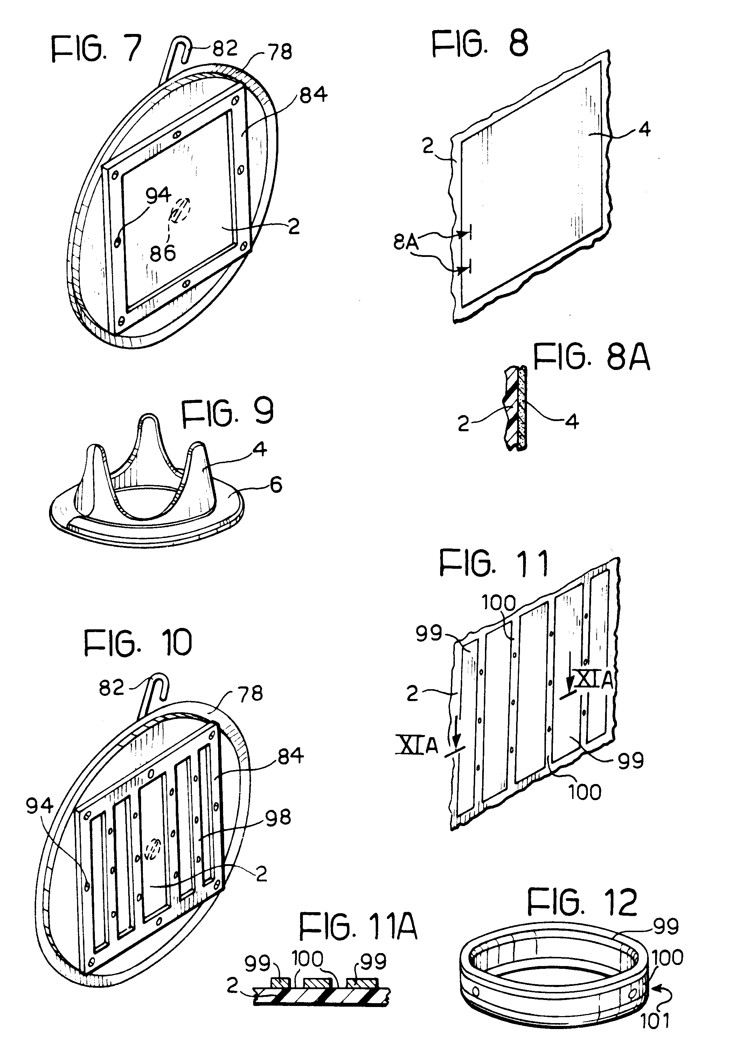

Referring first to the illustrative embodiments of the invention of FIGURES 1-9, there is shown a triode vacuum apparatus 10 (FIGURES 1-7) for coating a substrate 2 with a thin, uniform, dense coating or film 4 of biocompatible carbon firmly adherent to the substrate 2 (FIGURES 8 and 8A) for use in a prosthetic device 6 (FIGURE 9).

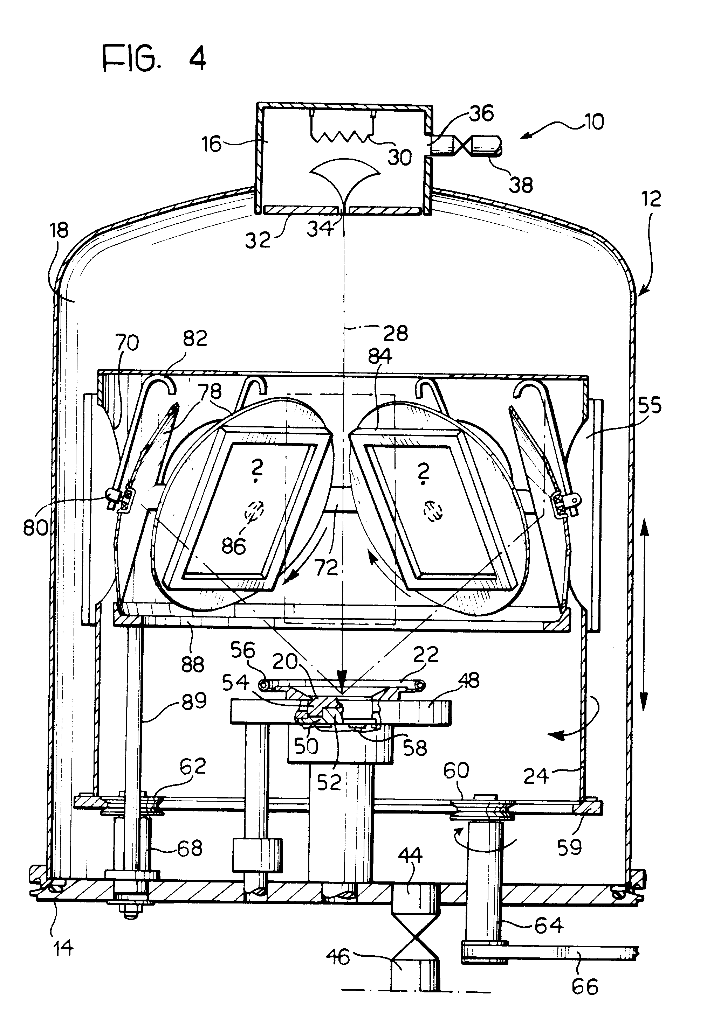

The apparatus 10 (FIGURES 1-6) includes a bell jar 12 mounted on a hydraulically operated telescoping arm 13 for moving the jar 12 to and from a base 14. The bell jar 12 has an upper ionization chamber 16 and a lower sputtering chamber 18. Positioned in the sputtering chamber 18 when the jar 12 is closed (FIGURES 1 and 4) is a target 20 which provides a source for the biocompatible carbon and which is circumscribed by an anode 22. Also within the closed sputtering chamber 18 is a removable support structure or housing 24 for the substrates 2 to be coated by the carbon (FIGURE 4). This illustrative apparatus is of the general type marketed by Balzers AG of Liechtenstein under Model BB 800 033 PE 7503.

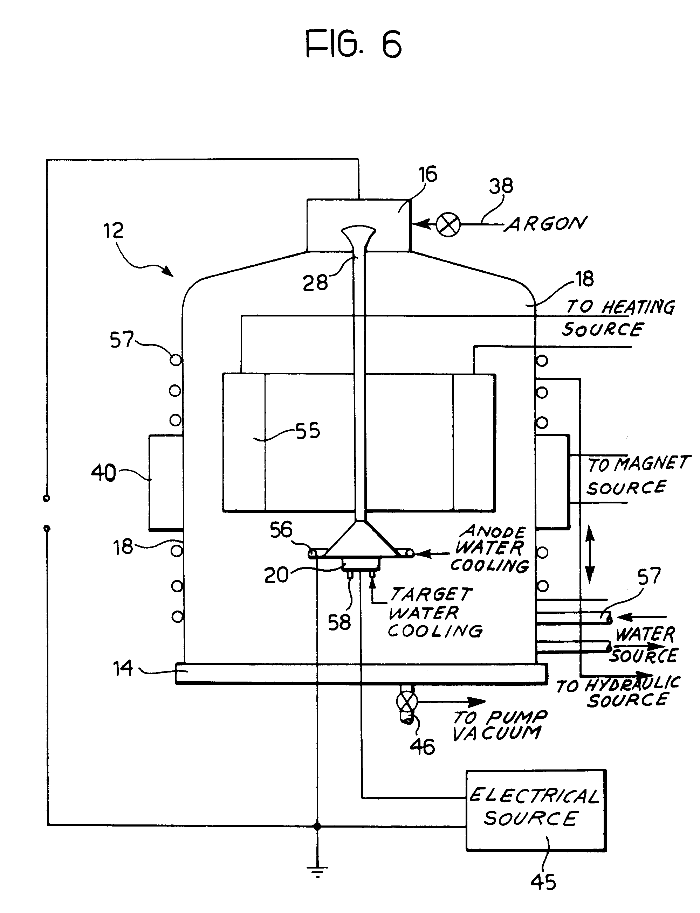

The ionization chamber 16 generates a plasma beam 28 (FIGURES 1 and 6) of concentrated ionized particles which are drawn to the target 20 for sputtering carbon onto the substrates 2. The chamber 16 includes a filament 30 at its upper end, an auxiliary anode 32 at its lower end having a central opening 34 therethrough and an intermediate inlet 36 for an inert gas, such as argon, which forms the plasma beam 28. The argon gas flows from a source (FIGURE 6) through a valve controlled conduit 38 connected to the inlet 36 and into the ionization chamber 16, wherein the heated filament 30 ionizes the argon into concentrated positively charged ions which are drawn through the opening 34 of the auxiliary anode 32 into sputtering chamber 18 to the target 20. As the argon ions are drawn through the sputtering chamber 18 they are collimated into the shape schematically illustrated in FIGURE 6 by a magnetic field coil 40 centrally positioned about the outer wall of the sputtering chamber 18. As desired gases in the sputtering chamber 18 can be removed by a vacuum pump connected to a vacuum outlet 44 in the base 14 via the valve controlled conduit 46 (FIGURE 3 and 4). Throughout the operation the vacuum pump maintains the sputtering chamber 18 at low pressures so that the environmental effect on the force and speed of the sputtered carbon is minimal.

As illustrated in FIGURE 4, the target 20 and the substrates 2 are spaced from the ionization chamber 16. Accordingly the heat generated in forming the plasma beam 28, in substance, does not reach or adversely affect the substrates 2.

The target 20 and anode 22 are mounted within and on a stand 48 affixed to the base 14 (FIGURES 2, 4 and 6). The target 20 is positioned within a cylindrical cavity 50 and is circumscribed by the annular anode 22.

The target 20 can comprise graphite or other carbon materials. In the illustrative embodiment (FIGURE 4), the target 20 is cylindrical and comprises a graphite base 52 upon which is positioned an outer layer 54 of pyrolytic carbon.

To produce the required adhesion of the deposited carbon without adversely affecting the substrate 2, high voltages and low currents are used for the target 20 by the electric source 45 (FIGURE 6). Also, the target 20 and anode 22 are water cooled. By so doing, heat from infrared radiation from the target 20 and anode 22 is minimized.

It has been found that the relatively high voltages, e.g., at least about 500 volts and preferably about 1000 to 3000 volts, provide the energy needed to properly adhere the sputtered carbon to the substrate and suitable deposition rate, while low direct currents, e.g., about 0.05 to 0.3 amps., minimize infrared and ultraviolet radiation. On the other hand, low voltage levels adversely affect the adhesion of the carbon to the substrate without meaningfully changing the generation of infrared radiation and high currents produce infrared and ultraviolet radiation that can adversely affect the substrates 2.

It also has been found that circulating cooling water from a source through an annular conduit 56 contiguous with the outer perimeter of the anode 22 and an annular conduit 58 beneath the cavity wall and in contact with the target 20 minimizes the generation of detrimental heat.

To further control the environment within the bell jar 12 and to enhance coating of the substrates 2, there are provided electric quartz heaters 55 mounted on the inner wall of the sputtering chamber 18 and about the mounted substrates 2, and a conduit 57 helically wrapped about the periphery of the outer wall of the sputtering chamber 18 through which water can be conveyed. The use of the heaters 55 and the conduit 57 to enhance the operation of the apparatus 10 will be explained in detail hereinafter.

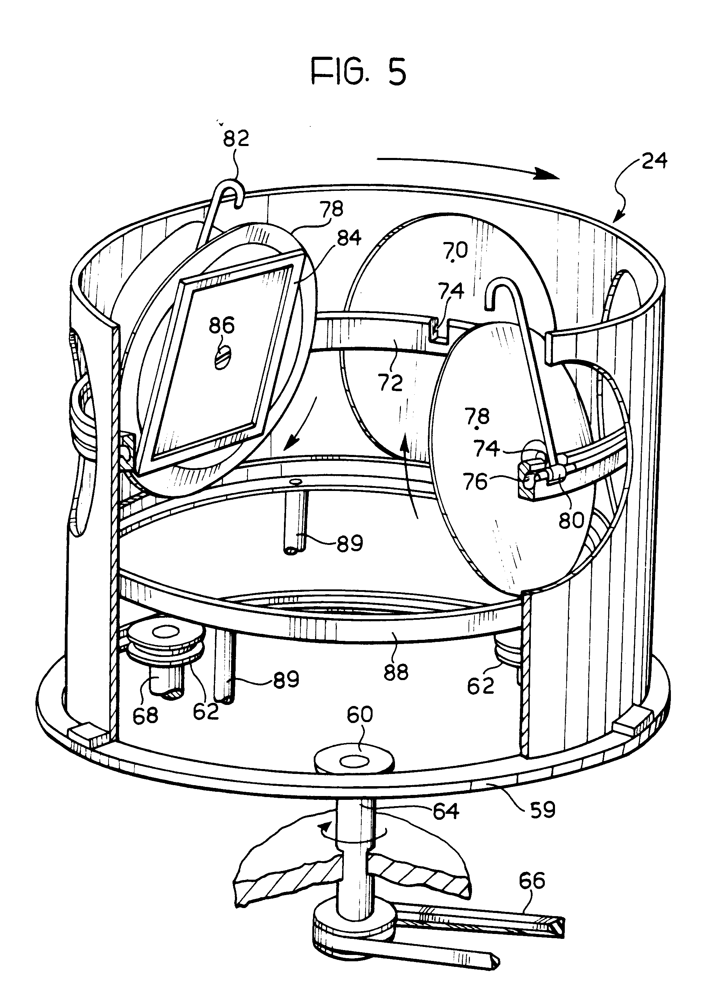

Removably and rotatably positioned within the sputtering chamber 18 is the internal housing 24 for the substrates 2 (FIGURES 4 and 5). The housing 24 and related structure are designed to facilitate the coating of the substrates 2 with a dense thin film of turbostratic carbon from the sputtered target 20. As will be presently described, such structure positions the substrates 2 relative to the target 20 and simultaneously rotates such substrates 2 at predetermined rates about their axis and the longitudinal axis of the bell jar 12 to optimize uniform coating thereon.

More particularly, the housing 24 is cylindrical and is supported at its lower end on a rotatable cylindrical ring 59 driven by a belt driven roller 60 and supported by idler rollers 62. The roller 60 is connected to the upper end of a shaft 64 rotated by a belt 66 which is driven by a motor (not shown). The idler rolls 62 are connected to the upper ends of shafts 68 which are connected at their lower ends to the base 14.

The housing 24 extends upwardly from the lower rotable ring 59, about the target 20 and anode 22 and into the upper portion of the sputtering chamber 18. In the upper portion of the housing 24 there are six equal spaced circular openings 70. A cylindrical rail 72 is secured to the inner wall of the housing 24 and traverses the openings 70. In the rail 72, there is a notch 74 centrally positioned in each opening 70 for allowing access to an annular slot 76 in the rear wall of the rail 72.

The described structure of the housing 24 is adapted to receive disks 78 which hold the substrates 2. Each disk 78 has a shaft 80 centrally connected to its convex rear wall which is removably seated in the notch 74 and adjacent slot 76, and a hanger 82 extending upwardly therefrom to facilitate carrying, holding and storing of the disks 78. Each disk 78 also includes a frame 84 on its concave front wall which is secured to the disk 78 by a screw or other means 86 and which is used to hold the substrate 2.

Within the housing 24 and at the base of the openings 70 is a separate stationary ring 88 having rods 89 depending therefrom which are connected at their lower ends to the base 14. As illustrated in engaging the rail 72, the disks 78 tilt slightly forward with their lower ends frictionally engaging the stationary ring 88. As a consequence, when the housing 24 is rotated within the sputtering chamber 18, the disks 78 simultaneously rotate about their axis and the longitudinal axis of the bell jar 12 for uniform coating of the substrate 2. In addition, the disks 78 are tilted forwardly toward the target 20 and positioned a predetermined distance therefrom to optimize uniform coating of the sputtered carbon onto the rotating substrates 2.

The disks 78 can be used to secure a variety of substrates 2. Referring to FIGURE 7 through 9 a rectangular shaped substrate 2 is secured in a correspondingly shaped frame 84 on a disk 78 by screws or other means 94. After forming the biocompatible carbon film 4 on the substrate 2 in accordance with the present invention, the coated substrate 2 is removed from the frame 84 and can be wrapped around and secured to a stent of a cardiac valve, for example, to form the prosthetic device 6 shown in FIGURE 9. In doing so, biocompatible carbon coated sutures, which are preferably also prepared in accordance with the present invention as hereinafter more particularly described, can be used.

Certain prosthetic devices require only portions of the substrate to be coated with a biocompatible carbon film. For example, cardiac valves may have only a portion of the valve exposed to physiological fluids, or it may be desirable to have portions of the prosthetic device attached by tissues after implantation. Those portions not exposed to tissues and fluids or portions which are desirably attached to tissues are masked prior to coating. In such instance the triode sputtering vacuum process of the invention will provide a carbon film only on the unmasked portions. More specifically, and as shown in FIGURE 10, the frame 84 has masking segments 98 overlaying the substrate 2. As a result, the present invention will provide a substrate 2 including coated and uncoated portions 99 and 100 (FIGURES 11 and 11A) which can be used to form a ring 101 (FIGURE 12) for a cardiac valve comprising a substrate 2 having coated and uncoated regions 99 and 100, respectively.

In addition to the valve components 6 and 101 just described, the present invention can be used for producing grafts and patches, tubular prostheses, sutures, catheters, otologic prostheses, tendon and ligament prostheses, dental implants, jaw replacements and other prosthestic devices. The prosthestic devices of the invention are especially useful as implants because they are biocompatible with body fluids and tissue. The implanted prosthetic devices of the invention resist physiological rejection and degradation. In each embodiment of the invention, the prosthetic device consists of or includes a substrate 2 with a thin, uniform, dense film or coating 4 and 99 of biocompatible carbon adherent thereto. See illustrative FIGURE 8A and 11A.

In the present invention, a wide variety of substrate materials can be used, however, the invention is particularly well suited for substrates comprising organic polymers having relatively low softening points. The substrates include DACRON which exhibits a change in elasticity at about 150°C. Therefore, it is preferred when coating DACRON to have the substrate exposed to temperatures no higher than the range of 80-90°C.

TEFLON is another suitable material which has low softening point substrate. TEFLON can withstand temperatures of about 250°C before softening. It is preferred to limit the temperature in proximity of a TEFLON substrate to no more than about 160-180°C.

Other low temperature materials which can be used for the substrate include silicone, polyacetal resins (such as those sold under the trademark DELRIN), polyurethane, low density polyethylene, and nonrefractory metals, such as Haynes 25 cobalt-chromium alloys, titanium and titanium alloys.

The thickness of the substrates will vary depending on the form of and type of material.

Substrates in the form of woven or knit fabrics, such as DACRON or TEFLON fabrics used in cardiac valves typically are from about 0.1 to 1.0 mm in thickness, and desirably 0.3 mm.

As stated, the carbon films or coatings of the invention deposited on the described substrates are biocompatible, continuous, uniform, dense and cover at least those portions of the substrates which are in contact with physiological fluids that can degrade uncoated substrates.

The carbon film preferably is thin within the range of about 0.2 to 0.8 micron, and not more than about 1.0 micron. Films having a thickness exceeding the preferred range tend to separate from the substrate and/or take too long to produce. Films below the preferred thickness may not uniformly cover the substrate surface.

The carbon film of the invention is dense, e.g., 2.2 gm/cm$^3$, and is turbostratic because this form of carbon is known to be biocompatible. The carbon film of the invention also strongly adheres to the substrate so that the coated substrate is able to repeatedly flex without separation of the film. The adhesion is tested in accordance with the ASTM 03359 standard procedure.

The process of the invention is admirably suited for forming the described biocompatible film on a wide variety of prosthetic devices consisting of or including coated substrates. To highlight the advantages of the invention, the following describes the process utilizing the illustrative embodiment of the apparatus 10 for forming the prosthetic device as shown in FIGURES 1 and 8, including low temperature substrates of medical grade DACRON and TEFLON.

Referring first to medical grade DACRON, the substrates 2 to be used are rectangular in shape as shown in FIGURE 8. Prior to initiating the operational phase of the process, the DACRON substrates are cleansed by a non-corrosive solvent.

The cleansed substrates 2 are mounted by the frame 84 to the disks 78 (FIGURE 7) and the loaded disks 78 are mounted on the rail 72 of the housing 24 (FIGURES 4 and 5). In so doing, the disks 78 tilt forward.

The bell jar 12 is raised from its base 14 by the hydraulically operated telescoping arm 13 and rotated on such arm 13 away from the base 14 as shown in FIGURE 3. The housing 24 with the assembled disks 78 are placed on the lower rotatable ring 59 and the lower ends of the tilted disks 78 frictionally engage the stationary rail 88 positioned within the housing 24. The bell jar 12 is then rotated over the housing 24 and lowered by the hydraulically operated telescoping arm 13 about the housing 24 and into sealing engagement with the base 14 (FIGURES 2 and 1).

The sputtering chamber 18 is then evacuated and maintained at low pressure levels ($10^{-4}$ mbar - $10^{-2}$ mbar and preferably $10^{-3}$ mbar) by a vacuum pump via the outlet 44 and conduit 46. This is done to enhance the adhesion of the sputtered and the deposited carbon on the substrates 2. At such low pressure levels, the sputtered carbon atoms maintain their high energy level.

The target 20 is cleansed by bombardment of the plasma beam 28, and the bell jar 12 is cleansed by removal of moisture and gaseous impurities. The cleansing of the bell jar 12 is accomplished by feeding hot water through the helical conduit 57 about the wall of the sputtering chamber 18 and such chamber 18 is evacuated by a vacuum pump to remove vapors and gases.

Also to enhance adhesion of the sputtered carbon on the substrates 2, the temperature within the sputtering chamber 18 is maintained at about 80°C by the use of the electric quartz lamps 55.

At the same time the filament 30 is operated at a current of about 80 to about 90 amperes, and preferably about 85 amperes, and argon gas is fed into the chamber 16 via conduits 38 and inlet 36, whereupon a plasma beam 28 is generated consisting of high energy positively charged ions. The plasma beam 28 is drawn from the ionization chamber 16 into the sputtering chamber 18 where it is focused or collimated by the magnetic field coil 40 and directed to the target 20. The coil 40 also serves to minimize exposure of the substrate 2 to secondary electrons. For such purposes, the coil 40 is operated at about 12 amperes.

The high energy argon ions of the plasma beam 28 bombard the target 20 causing carbon atoms to separate from the surface of the graphitic or pyrolytic target and flow in the acute angular direction schematically shown in FIGURE 4 at substantial speeds onto the substrates 2. The substrates 2 are rotated about the longitudinal axis of the bell jar 12 and about their own axis to enhance uniform deposition of carbon. Typically, rotation about the jar 12 is at the rate of about 4 to 5 revolutions about their axis for each revolution about the jar 12.

During operation, the target 20 is maintained at high voltages and low direct currents, such as 3000 volts and 0.3 amps, by the electric source. The high voltages help generate a suitable number of sputtered carbon atoms of the desired high energy while the low currents minimize the generation of infrared and ultraviolet radiation. Also, during operation the anode 22 and target 20 are cooled by water flowing at about 20°C through their respective conduits 56 and 58 to minimize the generation of heat.

To further reduce the amount of undesirable heat at the substrate 2, the ionization chamber 16 is at the upper end of the sputtering chamber 18 and spaced a considerable distance from the substrate 2. In this illustrative example, the ionization chamber 16 is about 25 cm from the substrates 2.

The described process is continued until the carbon film 4 uniformly deposited on the DACRON substrates is about 0.3 microns in thickness. To achieve this thickness, the process is operated for about 4.5 hours.

After the desired thickness of biocompatible carbon film is obtained, the argon gas feed is shut off. After the pressure in the sputtering chamber 18 is raised to atmospheric pressure, the bell jar 12 is opened and the housing 24 is removed from the base 14. Thereafter, the disks 78 are removed from the housing 24 and the coated substrates 2 are removed from the frames 84.

The prosthetic devices thereby obtained include the desired carbon film 4 on the substrate 2 with the necessary adhesion therebetween and without adversely affecting the substrate.

In coating substrates 2 of TEFLON essentially the same process is employed. However, to obtain optimum adhesion of the carbon film 4, it has been found that a temperature gradient at the substrates 2 within the sputtering chamber 18 is desirable. With TEFLON substrates, the quartz electric lamps 55 are operated so as to heat the environment at the substrates 2 to about 180°C until about one-sixth of the total desired thickness of the carbon film (0.3 microns) is deposited on the TEFLON substrates. Thereafter, heating of the substrate is terminated and the temperature in and about the substrates 2 is cooled down to and maintained at about 40°C. This is accomplished by shutting off the quartz electric lamps 55 and conveying cooling water through the helical conduit 57 about the outer wall of the sputtering chamber 18. Deposition of carbon to the full thickness of the deposited carbon was carried out under the same conditions already described, except that the anode current was reduced to about 0.1 amps. The operational time was about 8 hours.

The prosthetic devices obtained by the process produced the desired carbon films 4 on the TEFLON substrates 2 with the required adhesion therebetween and without adversely affecting the TEFLON.

Thus far there has been described, in some detail, the coating of flat substrates 2 for use in prosthetic devices. It is to be understood that the present invention can be used to provide biocompatible carbon films on a variety of prosthetic devices of different configurations, including tubular structures (e.g., vascular tubing and catheters) and yarns (e.g., sutures).

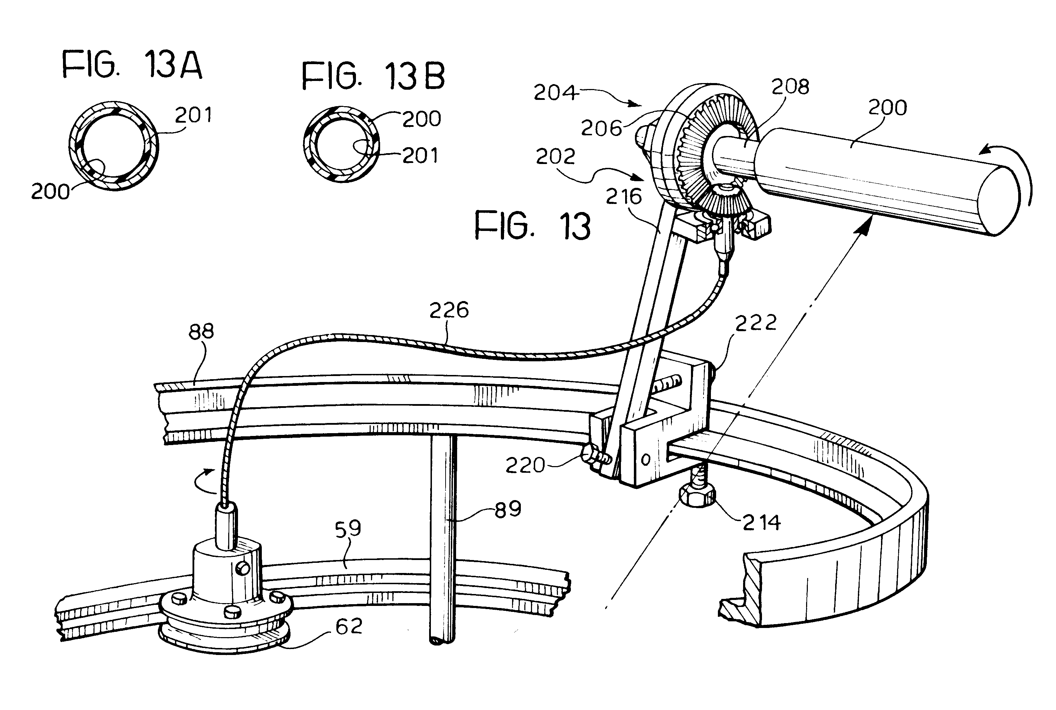

In FIGURE 13, for example, vascular tubing 200 is coated with a carbon film 201 of the present invention (FIGURE 13A). The illustrative apparatus 202 includes a drive assembly 204 comprising a gear mechanism 206 which rotates a spindle 208 having the tubular substrate 200 thereon. The drive assembly 204 is secured to the stationary rail 88 by a clamp 208 and bolt 214. A bar 216 of the assembly 204 is adjustably secured at one end to the clamp 210 by bolts 220 and 222 and at its other end to the gear mechanism 206.

The gear mechanism 206 is rotated by a rotable flexible cable 226 connected at one end to the gear mechanism 206 and its other end to the previously described idler roller. Rotation of the ring 59 by the drive roller 60 (FIGURE 6) causes the idler roller 62 to rotate cable 226 which, in turn, rotates the gear mechanism 206. In so doing, the gear mechanism 206 rotates the spindle 208 and tubing 200 thereon.

In operation, the tubular substrate 200 is rotated during the sputtering process whereby sputtered carbon atoms from the target 20, as already described and illustrated (FIGURES 1-6), impinge on the tubular substrate 200 until a uniform thin biocompatible carbon film 201 is obtained. If desired, the coated exterior surface of the tubing 200 can be inverted by inserting the tubing within itself to provide an inert coated interior surface. Also, this technique can be used for coating both surfaces of the tubing 200 by simply remounting the inverted tubing 200 on the spindle 208 and repeating the process to coat its "new" uncoated exterior surface.

In accordance with the described process tubing of DACRON was coated with the biocompatible carbon film 201 of the invention by rotating the tubing 200 about 5 revolutions per minute in about 9 hours under conditions similar to the described conditions for coating the substrate 2 of DACRON.

Biocompatible carbon coated yarns, such as sutures, also can be produced in accordance with the present invention.

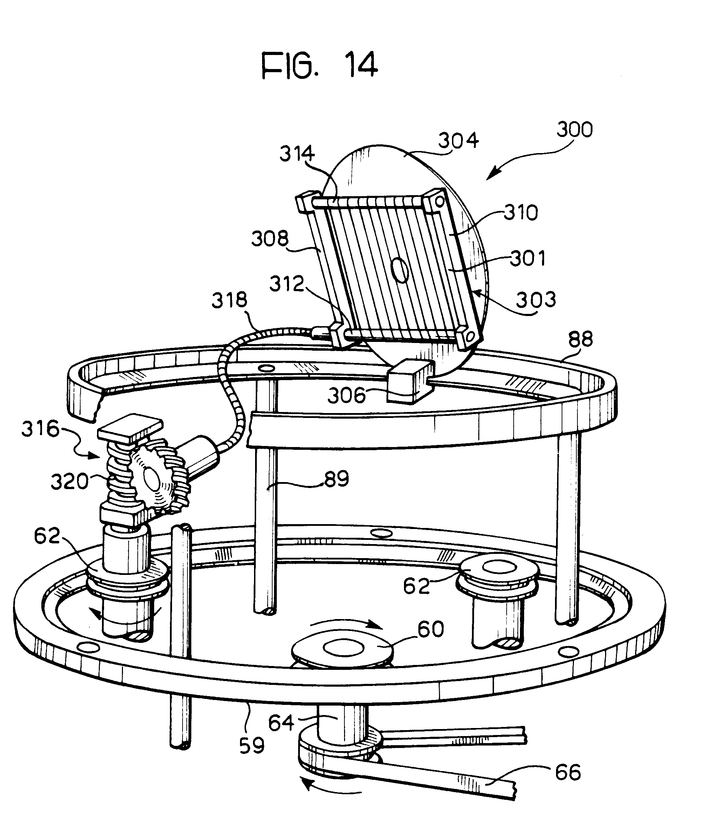

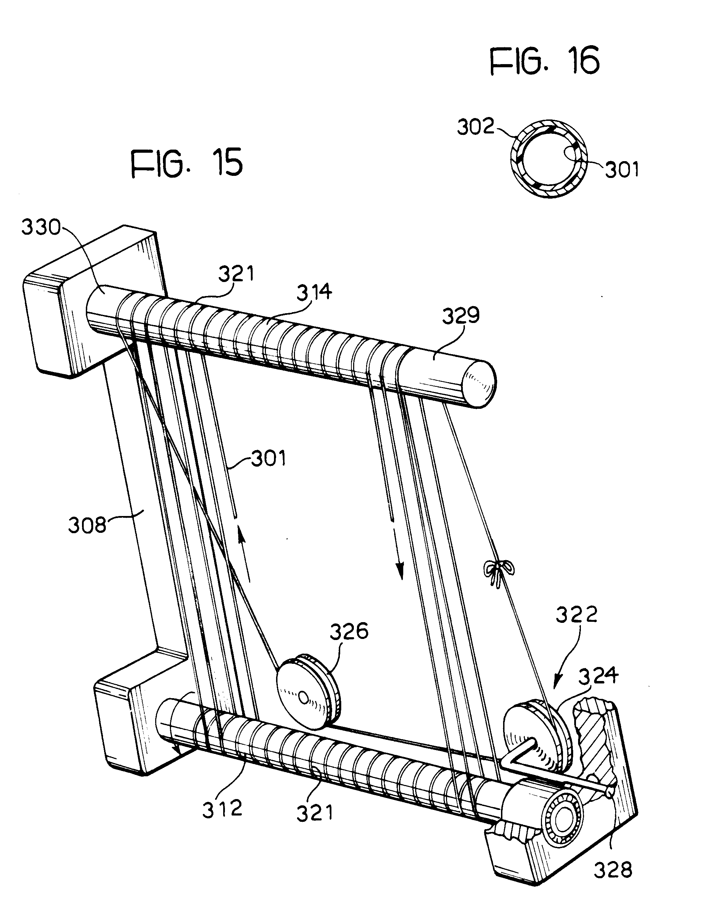

As shown in FIGURES 14, 15 and 16 there is provided a device 300 for coating suture yarn 301 with a uniform, thin and dense adherent film of biocompatible carbon 302 (FIGURE 16). The device 300, includes a frame 303 attached to a diSk 304 secured by a clamp 306 to the stationary rail 88. The frame 300 includes vertical supports 308 and 310 with transverse rods 312 and 314 therebetween.

The lower rod 312 is rotated by a drive assembly 316 including a rotatable flexible cable 318 connected at one end to the lower rod 312 for rotation thereof, and at the other end to a gear mechanism 320, which, in turn, is rotatably connected to the previously described idler roller 62. As the lower rotatable ring 59 is rotated by the drive roller 60, such rotation is transferred through the idler roller 62, gear mechanism 320 and flexible cable 318 to the lower rod 312.

The rods 312 and 314 include grooves 321 therein for receiving progressive loops of the suture yarn 301 (FIGURE 15) as it is rotated by the rod 312. As the rod 312 is so rotated, carbon is sputtered from the target 20, as previously described (FIGURES 1-6), and is deposited on and firmly adheres to the moving yarn 301 to form the biocompatible carbon film 302 (FIGURE 16). The grooves 321 are arranged in spaced apart relationship along the rods 312 and 314. As the yarn 301 moves from one groove 321 to another, it is slightly twisted thereby progressively exposing uncoated portions of the yarn to the sputtered carbon.

There also is provided a threading assembly 322, including rotatable pulleys 324 and 326 supported by supports 328 and frame 303.

When the suture yarn 301 comes off the far end 329 of the rod 314 after being coated, it is engaged by the threading assembly 322 and transported to the near end 330 of the bar 314 for further coating as described above. The threading assembly 322 thereby provides means for coating the yarn 301 continuously until the desired coating thickness is obtained.

Following the steps of the described process, suture yarn 301 of DACRON was coated with the biocompatible carbon film 302 of the invention by progressively looping the yarn 301 over the device 300 for about 6.5 hours.

Thus, the present invention includes a wide variety of prosthetic devices, as well as methods of and apparatus for forming such devices. In its broader aspects, the invention is not limited to the specific described embodiments and departures may be made therefrom within the scope of the accompanying claims without departing from the principles of the invention and without sacrificing its chief advantages.

## Claims

1. A method of forming a prosthetic device having a substrate and a coating (4) of biocompatible carbon material deposited on said substrate by sputtering a carbon target (20) at a given voltage and current (20, 22) characterised in that sputtering is effected as d.c. sputtering operating the carbon target (20) at least at about 500 volts and at a current which is not more than about 0.3 amps.

2. A method according to claim 1, characterised in that the target (20) is operated at about 1000 to 3000 volts and a current of about 0.05 to 0.3 amps.

3. A method according to claims 1 or 2 characterised in that the substrate is flat (2) and is rotated (24) about the carbon target (20) and about itself for uniform distribution of the sputtered carbon thereon.

4. A method according to claim 1 or 2 characterised in that the substrate is tubular (200) and is rotated (204, 206, 208) about its axis for uniform distribution of the sputtered carbon thereon.

5. A method according to claims 1 or 2, characterised in that the substrate is suture yarn (301) and is rotated about a frame (303) for uniform distribution of the sputtered carbon thereon.

6. A method according to claims 1 or 2, characterised in that portions of the substrate or material are masked (98) so that the sputtered carbon is deposited only on unmasked portions.

7. A method according to any of the preceding claims, characterised in that the substrate is a polyester resin and the temperature at the substrate (2) is maintained at about 80°C to enhance the adhesion of the sputtered carbon (4) to the substrate.

8. The method according to any of claims 1 to 6, characterised in that the substrate (2) is polytetrafluoroethylene and the temperature at the substrate is maintained at about 180°C for about one-sixth of the desired carbon coating thickness, and then at about 40°C for the balance of the coating (4) of carbon, to thereby enhance the adhesion of the sputtered carbon to the substrate.

9. A method according to any claims 1 to 8, characterised in that the carbon sputtered (20) onto the substrate has a thickness of not more than about 1.0 micron.

10. A method according to claims 1 or 2, characterised in that the carbon target (20) is sputtered in an atmosphere having a pressure of about $10^{-4}$ mbar to about $10^{-2}$ mbar.

11. Apparatus for depositing a film (4) of biocompatible carbon firmly adherent to a substrate of a prosthetic device including a carbon target (20) positioned within a chamber (18), means (16) for sputtering said carbon target (20) to sputter carbon atoms therefrom, holding means (24; 208; 303) mounted in said chamber (18) for holding a substrate to form a firmly adherent carbon coating thereon, pressurising means (44, 46) within and about said chamber for operating said chamber (18) at a low pressure, and electrical means (45) connected to said carbon target (20) for operating the target (20) itself at a given voltage and current, characterised in that said electrical means comprises an electrical source (45) for maintaining the target (20) under d.c. sputtering conditions at a sputtering voltage of at least about 500 volts and at a current of not more than about 0.3 amps.

12. Apparatus according to Claim 11, characterised in that heating means (55) are provided in said chamber (18) and in proximity of said holding means (24) for heating the substrate (2) to desired levels to enhance the adhesion of sputtered carbon (20) to the substrate (2).

13. Apparatus according to claim 11 or 12 wherein a plurality of substrates (2) are simultaneously coated with sputtered biocompatible carbon, characterised in that said holding means (24) comprises:
    rotatable means (59) mounted at the bottom of said chamber (18);
    a housing (24) removably mounted on and rotatable with said rotatable means which extends upwardly therefrom and about said carbon target (20),
    support means (78) for each substrate (2) releasably connected to said housing (24) for holding a substrate (2) thereon during sputtering of the carbon (20), wherein said support means (78) rotates with said housing (24) about said carbon target (20) and is rotatable, at the same time, about its own axis,
    drive means (60) acting on said rotatable means (59) for rotation thereof, and

rotation means (88) positioned in said chamber (18) for frictionally engaging each of said support means (78) to effect rotation thereof about its own axis as said housing (24) rotates with said support means about said carbon target (20).

14. Apparatus according to claim 13, characterised in that each of said support means (78) for the substrate comprises:

(a) a disk (78) having a convex rear wall and a concave front wall; and
(b) a frame (84) mounted on the concave front wall of said disk for releasably connecting the substrate (2) thereto, and wherein
(c) said frictionally engaging rotation means is an annular rail (88) engaged by the bottom of each of said disks.

15. Apparatus according to claim 14, characterised in that said substrates (2) have a flat cross-sectional shape.

16. Apparatus according to claim 11 or 12, for coating biocompatible carbon on a tubular substrate (200) of a prosthetic device, characterised in that said holding means for the substrate comprises:

a rotatable spindle (208) mounted in said chamber (18) in the path of the sputtered carbon for mounting the tubular substrate (200) thereon, and

drive means (204, 206) positioned in said chamber (18) for rotating said spindle (208) so that sputtered carbon is uniformly deposited on the exposed surface of the rotating tubular substrate (200).

17. Apparatus according to claim 11 or 12 for coating biocompatible carbon on a substrate of suture yarn (301), characterised in that said holding means for the substrate of suture yarn comprises:

a frame (303) mounted in said chamber (18) in the path of the sputtered carbon, including a pair of transversely spaced rods (312, 314) having a plurality of grooves (321) therein for receiving the suture yarn (301) as it is rotated on said rods (308, 310) wherein said grooves (321) are aligned between rods (312, 314) in such a manner that the yarn (301) turns slightly as it continuously travels from one grove (321) to another therebetween,

motor means (316) connected to one (312) of said rods for rotation thereof, and

threading means (322) connected to said frame (303) for transferring the suture yarn (301) coming off one end of one (314) of said transverse rods to thereby continuously rotate said suture yarn (301) until it is coated to the desired thickness with firmly adherent biocompatible sputtered carbon.

## Revendications

1. Procédé de fabrication d'une prothèse, pourvue d'un substrat et d'un revêtement (4) en matériau au carbone biocompatible, déposé sur ledit substrat par pulvérisation d'une cible au carbone (20), à une tension et un courant (20, 22) donnés, caractérisé en ce que la pulvérisation est effectuée sous forme de pulvérisation à courant continu agissant sur la cible au carbone (20) avec une tension d'au moins à peu près 500 volts et un courant qui ne dépasse pas 0,3 ampères.

2. Procédé selon la revendication 1, caractérisé en ce qu'il agit sur la cible (20) avec une tension d'à peu près 1000 à 3000 volts et un courant d'à peu près 0,05 à 0,3 ampères.

3. Procédé selon la revendication 1 ou 2, caractérisé en ce que le substrat est plat (2) et est entraîné en rotation (24) autour de la cible au carbone (20) et sur lui-même, pour assurer une distribution uniforme du carbone pulvérisé sur ce dernier.

4. Procédé selon la revendication 1 ou 2, caractérisé en ce que le substrat est tubulaire (200) et est entraîné en rotation (204, 206, 208) autour de son axe, pour assurer une distribution uniforme du carbone pulvérisé sur ce dernier.

5. Procédé selon la revendication 1 ou 2, caractérisé en ce que le substrat est un fil de suture (301) et est entraîné en rotation autour d'un cadre (303), pour assurer une distribution uniforme du carbone pulvérisé sur ce dernier.

6. Procédé selon la revendication 1 ou 2, caractérisé en ce que des parties du substrat ou matériau sont masquées (98), de sorte que le carbone pulvérisé est déposé uniquement sur les parties non masquées.

7. Procédé selon l'une quelconque des revendications précédentes, caractérisé en ce que le substrat est une résine au polyester et la température appliquée sur le substrat (2) est maintenue à peu près à 80°C, pour améliorer l'adhésion du carbone pulvérisé (4) au substrat.

**8.** Procédé selon l'une quelconque des revendications 1 à 6, caractérisé en ce que le substrat (2) est du polytétrafluoroéthylène et la température appliquée sur le substrat est maintenue à peu près à 180°C, pour obtenir à peu près un sixième de l'épaisseur de revêtement au carbone souhaité et ensuite, à peu près 40°C, pour obtenir l'épaisseur restante du revêtement (4) au carbone, afin d'améliorer l'adhésion du carbone pulvérisé sur le substrat.

**9.** Procédé selon l'une quelconque des revendications 1 à 8, caractérisé en ce que le carbone pulvérisé (20) sur le substrat présente une épaisseur ne dépassant pas à peu près 1,0 micron.

**10.** Procédé selon la revendication 1 ou 2, caractérisé en ce que la cible au carbone (20) est pulvérisée dans une atmosphère d'une pression d'à peu près $10^{-4}$ mbar à peu près à $10^{-2}$ mbar.

**11.** Appareil pour déposer un film (4) de carbone biocompatible, adhérant rigidement à un substrat d'une prothèse, comprenant une cible au carbone (20) placée à l'intérieur d'une chambre (18), un moyen (16) pour pulvériser ladite cible au carbone (20) en atomes de carbone de pulvérisation à partir de cette dernière, un moyen de maintien (24; 208; 303) monté dans ladite chambre (18) pour maintenir un substrat, afin de former un revêtement au carbone adhérant rigidement sur ce dernier, un moyen de pressurisation (44, 46), situé à l'intérieur et autour de ladite chambre, pour actionner ladite chambre (18) à une faible pression, et un moyen électrique (45), relié à ladite cible au carbone (20) pour actionner la cible (20) même, à une tension et un courant donnés, caractérisé en ce que ledit moyen électrique comprend une source électrique (45), pour maintenir la cible (20) dans des conditions de pulvérisation à courant continu, c'est-à-dire à une tension de pulvérisation d'au moins à peu près 500 volts et un courant ne dépassant pas à peu près 0,3 ampères.

**12.** Appareil selon la revendication 11, caractérisé en ce que des moyens de chauffage (55) sont prévus dans ladite chambre (18) et à proximité dudit moyen de maintien (24), pour chauffer le substrat (2) à des niveaux souhaités, afin d'améliorer l'adhésion du carbone pulvérisé (20) sur le substrat (2).

**13.** Appareil selon la revendication 11 ou 12, dans lequel une pluralité de substrats (2) sont simultanément revêtus par du carbone biocompatible pulvérisé, caractérisé en ce que ledit moyen de maintien (24) comprend:

un moyen rotatif (59) monté au fond de ladite chambre (18),

un boîtier (24) monté de façon détachable et susceptible de tourner avec ledit moyen rotatif, qui s'étend vers le haut de ce dernier, autour de ladite cible au carbone (20),

un moyen de support (78) pour chaque substrat (2), relié, de façon détachable, audit boîtier (24), pour maintenir un substrat (2) sur ce dernier durant la pulvérisation du carbone (20), dans lequel ledit moyen de support (78) tourne avec ledit boîtier (24), autour de ladite cible au carbone (20) et, en même temps, est susceptible de tourner autour de son propre axe,

un moyen d'entraînement (60) agissant sur ledit moyen rotatif (59) pour le faire tourner et

un moyen de rotation (88) placé dans ladite chambre (18), pour venir en contact de frottement avec chacun desdits moyens de support (78), pour le faire tourner autour de son propre axe, lorsque ledit boîtier (24) tourne avec ledit moyen de support, autour de ladite cible au carbone (20).

**14.** Appareil selon la revendication 13, caractérisé en ce que chacun des moyens de support (78) pour le substrat comprend:

(a) un disque (78) présentant une paroi arrière convexe et une paroi avant concave et

(b) un cadre (84) monté sur la paroi avant concave dudit disque, pour relier, de façon détachable, le substrat (2) à ce dernier, et dans lequel

(c) ledit moyen de rotation en contact de frottement est un rail annulaire (88), en contact avec le verso de chacun desdits disques.

**15.** Appareil selon la revendication 14, caractérisé en ce que lesdits substrats (2) présentent une section transversale de forme plate.

**16.** Appareil selon la revendication 11 ou 12, pour revêtir du carbone biocompatible sur un substrat tubulaire (200) d'une prothèse, caractérisé en ce que ledit moyen de maintien pour le substrat comprend:

une broche rotative (208) montée dans ladite chambre (18), dans le chemin du carbone pulvérisé, pour monter le substrat tubulaire (200) sur cette broche et

un moyen d'entraînement (204, 206) placé dans ladite chambre (18), pour entraîner ladite broche (208) en rotation, de façon que le carbone pulvérisé soit déposé,de façon uniforme, sur la surface exposée du substrat tubulaire rotatif (200).

17. Appareil selon la revendication 11 ou 12 pour revêtir du carbone biocompatible sur un substrat en fil de suture (301), caractérisé en ce que ledit moyen de maintien pour le substrat en fil de suture comprend:

un cadre (303) monté dans ladite chambre (18), dans le chemin du carbone pulvérisé, comportant un couple de tiges espacées transversalement (312, 314), présentant, en son sein, une pluralité de rainures (321) destinées à loger le fil de suture (301), lorsqu'il est entraîné en rotation sur lesdites tiges (308, 310), dans lequel lesdites rainures (321) sont alignées entre les tiges (312, 314), de façon que le fil (301) tourne légèrement tout en passant, de façon continue, d'une rainure (321) à une autre, entre ces tiges,

un moyen moteur (316) relié à l'une (312) desdites tiges, pour la faire tourner, et

un moyen de filetage (322) relié audit cadre (303), pour le transfert du fil de suture (301) arrivant à la fin de l'une (314) desdites tiges transversales, afin d'entraîner en rotation continue ledit fil de suture (301), jusqu'à ce qu'il soit revêtu de l'épaisseur souhaitée de carbone biocompatible pulvérisé adhérant rigidement.

**Patentansprüche**

1. Verfahren zur Herstellung einer Protheseneinrichtung, die eine Trägerschicht und eine Beschichtung (4) aus einem biokompatiblen Kohlenstoffmaterial besitzt, die auf der Trägerschicht durch die Zerstäubung einer Kohlenstoff-Fangelektrode (20) bei gegebener Spannung und Strom (20, 22) abgeschieden wird, dadurch gekennzeichnet, daß die Zerstäubung als Gleichspannungs-Zerstäubung ausgeführt wird, wobei die Kohlenstoff-Fangelektrode (20) mit zumindest etwa 500V und mit einem Strom betrieben wird, der nicht größer als etwa 0,3A ist.

2. Verfahren gemäß Anspruch 1, dadurch gekennzeichnet, daß die Fangelektrode (20) mit etwa 1000 bis 3000V und einem Strom von etwa 0,05 bis 0,3A betrieben wird.

3. Verfahren gemäß Anspruch 1 oder 2, dadurch gekennzeichnet, daß die Trägerschicht eben (2) ist und um die Kohlenstoff-Fangelektrode (20) sowie um sich selbst in Drehung versetzt wird (24), um den zerstäubten Kohlenstoff darauf gleichförmig zu verteilen.

4. Verfahren gemäß Anspruch 1 oder 2, dadurch gekennzeichnet, daß die Trägerschicht schlauchförmig (200) ausgebildet ist und um ihre Achse in Drehung versetzt wird (204, 206, 208), um den zerstäubten Kohlenstoff darauf gleichförmig zu verteilen.

5. Verfahren gemäß Anspruch 1 oder 2, dadurch gekennzeichnet, daß die Trägerschicht ein Nähgarn (301) ist, wobei sie in einem Rahmen (303) in Drehung versetzt wird, um den zerstäubten Kohlenstoff darauf gleichförmig zu verteilen.

6. Verfahren gemäß Anspruch 1 oder 2, dadurch gekennzeichnet, daß Bereiche der Trägerschicht oder des Materials abgedeckt (98) werden, so daß der zerstäubte Kohlenstoff nur auf den nichtabgedeckten Bereichen abgeschieden wird.

7. Verfahren gemäß jedem der bisherigen Ansprüche, dadurch gekennzeichnet, daß die Trägerschicht ein Polyesterharz ist, und daß die Temperatur auf der Trägerschicht (2) bei etwa 80°C gehalten wird, um die Adhäsion des zerstäubten Kohlenstoffs (4) an der Trägerschicht zu verbessern.

8. Verfahren gemäß jedem der Ansprüche 1 bis 6, dadurch gekennzeichnet, daß die Trägerschicht (2) Polytetrafluoräthylen ist, und daß die Temperatur auf der Trägerschicht für etwa ein Sechstel der gewünschten Dicke auf etwa 180°C der Kohlenstoffbeschichtung und dann auf etwa 40°C gehalten wird, um die Beschichtung (4) aus Kohlenstoff abzugleichen, um dadurch die Adhäsion des zerstäubten Kohlenstoffs an der Trägerschicht zu verbessern.

9. Verfahren gemäß jedem der Ansprüche 1 bis 8, dadurch gekennzeichnet, daß der auf die Trägerschicht zerstäubte (20) Kohlenstoff eine Dicke von nicht mehr als 1,0 Mikron besitzt.

10. Verfahren gemäß Anspruch 1 oder 2, dadurch gekennzeichnet, daß die Kohlenstoff-Fangelektrode (20) in einer Atmosphäre zerstäubt wird, die einen Druck von etwa $10^{-4}$mbar bis etwa $10^{-2}$mbar besitzt.

11. Vorrichtung zur Abscheidung einer Schicht (4) aus biokompatiblem Kohlenstoff, die fest an der Trägerschicht einer Protheseneinrichtung anhaftet, wobei die Vorrichtung eine Kohlenstoff-Fangelektrode (20), die in einer Kammer (18) angeordnet ist, eine Einrichtung (16), um die Kohlenstoff-Fangelektrode (20) zu zerstäuben, um Kohlenstoffatome davon zu zerstäuben, eine Halterung (24; 208; 303), die auf der Kammer (18) befestigt ist, um eine Trägerschicht zu halten, um darauf eine fest anhaftende Kohlenstoffbeschichtung auszubilden, eine Druckeinrichtung (44, 46) innerhalb der und um die Kammer, um die Kammer (18) bei einem niedrigen Druck zu betreiben, sowie eine elektrische Einrichtung (45) aufweist, die mit der Kohlenstoff-Fangelektrode (20) verbunden ist, um die Fangelektrode (20) bei einer gegebenen Spannung und Strom zu betreiben, dadurch gekennzeichnet, daß die elektrische Einrichtung eine elektrische Quelle (45) enthält, um die Fangelektrode (20) bei einer Gleichspannungs-Zerstäubung auf einer Zerstäubungsspannung von zumindest etwa 500V und einem Strom von nicht mehr als etwa 0,3A zu halten.

12. Vorrichtung gemäß Anspruch 11, dadurch gekennzeichnet, daß eine Heizeinrichtung (55) in der Kammer (18) und in der Nähe der Halterung (24) vorgesehen ist, um die Trägerschicht (2) auf ein gewünschtes Niveau zu erwärmen, um die Adhäsion des zerstäubten Kohlenstoffs (20) an der Trägerschicht (2) zu verbessern.

13. Vorrichtung gemäß Anspruch 11 und 12, wobei eine Vielzahl von Trägerschichten (2) gleichzeitig mit zerstäubtem, biokompatiblem Kohlenstoff beschichtet wird, dadurch gekennzeichnet, daß die Halterung (24) enthält:
    eine drehbare Einrichtung (59), die am Boden der Kammer (18) befestigt ist;
    ein Gehäuse (24), das auf der drehbaren Einrichtung abnehmbar und drehbar mit dieser befestigt ist, wobei es sich davon nach oben und um die Kohlenstoff-Fangelektrode (20) verläuft;
    eine Halteeinrichtung (78) für jede Trägerschicht (2), die abnehmbar mit dem Gehäuse (24) verbunden ist, um eine Trägerschicht (2) darauf während der Zerstäubung des Kohlenstoffs (20) zu halten, wobei sich die Halteeinrichtung (78) mit dem Gehäuse (24) um die Kohlenstoff-Fangelektrode (20) dreht und gleichzeitig um ihre eigene Achse drehbar ist,
    eine Antrieb (60), der auf die drehbare Einrichtung (59) wirkt, um diese in Drehung zu versetzen, und
    eine Dreheinrichtung (88), die in der Kammer (18) angeordnet ist, um reibungsmäßig in jede Halterungseinrichtung (78) einzugreifen, um eine Drehung davon um die eigene Achse hervorzurufen, wenn sich das Gehäuse (24) mit der Halteeinrichtung um die Kohlenstoff-Fangelektrode (20) dreht.

14. Vorrichtung gemäß Anspruch 13, dadurch gekennzeichnet, daß jede der Halteeinrichtungen (78) für die Trägerschicht enthält:
    (a) eine Scheibe (78), die eine konvexe Rückwand und konkave Vorderwand besitzt; und
    (b) einen Rahmen (84), der an der konkaven Vorderwand der Scheibe befestigt ist, um die Trägerschicht (2) abnehmbar zu verbinden, und wobei
    (c) die reibungsmäßig eingreifende Dreheinrichtung eine Ringschiene (88) ist, in die der Boden jeder Scheibe eingreift.

15. Vorrichtung gemäß Anspruch 14, dadurch gekennzeichnet, daß die Trägerschichten (2) einen flachen Querschnitt besitzen.

16. Vorrichtung gemäß Anspruch 11 oder 12, um biokompatiblen Kohlenstoff auf eine schlauchförmige Trägerschicht (200) einer Protheseneinrichtung zu beschichten, dadurch gekennzeichnet, daß die Halterung für die Trägerschicht enthält:
    eine drehbare Welle (208), die in der Kammer (18) in der Bahn des zerstäubten Kohlenstoffs befestigt ist, um darauf die schlauchförmige Trägerschicht (200) anzubringen, und
    eine Antriebseinrichtung (204, 206), die in der Kammer (18) angeordnet ist, um die Welle (208) in Drehung zu versetzen, so daß der zerstäubte Kohlenstoff gleichförmig auf der freiliegenden Fläche der rotierenden, schlauchförmigen Trägerschicht (200) abgeschieden wird.

17. Vorrichtung gemäß Anspruch 11 oder 12, um biokompatiblen Kohlenstoff auf der Trägerschicht eines Nähgarns (301) zu beschichten, dadurch gekennzeichnet, daß die Halterung für die Trägerschicht des Nähgarns enthält:
    einen Rahmen (303), der in der Kammer (18) in der Bahn des zerstäubten Kohlenstoffs befestigt ist, wobei er zwei in Querrichtung beabstandete Stangen (312, 314) aufweist, die eine Vielzahl von Rillen (321) besitzen, die darin ausgebildet sind, um das Nähgarn (301) aufzunehmen, wenn dieses um die Stangen (308, 310) gedreht wird, wobei die Rillen (321) zwischen Stangen (312, 314) so ausgerichtet sind, daß sich das Garn (301) etwas verdreht, wenn es fortlaufend von einer Rille (321) zu einer anderen läuft,
    einen Motor (316), der mit einer (312) der Stangen verbunden ist, um sie in Drehung zu versetzen, und

eine Verdrillungseinrichtung (322), die mit dem Rahmen (303) verbunden ist, um das von einem Ende von einer (314) der Querstangen kommende Nähgarn (301) zu transportieren, um dadurch das Nähgarn (301) fortlaufend zu drehen, bis es mit einem fest anhaftenden, biokompatiblen, zerstäubten Kohlenstoff auf die gewünschte Dicke beschichtet ist.

FIG. 1

FIG. 2

ARGON

TO HYDRAULIC SOURCE

VACUUM OUTLET

ELECTRICAL
SOURCE

FIG. 3

COOLING
LINE

14

FIG. 4

# FIG. 5

# FIG. 6

FIG. 7

82 78 84

94

86

2

FIG. 8

2 4

8A

FIG. 8A

2 4

FIG. 9

4

6

FIG. 10

82 78

84

94

98

2

FIG. 11

100

99

2

XIA

XIA

99

100

FIG. 11A

99 100 99

2

FIG. 12

99

100

101

18

FIG. 13A

FIG. 13B

FIG. 13

EP 0 224 080 B1

# FIG. 14

FIG. 16

FIG. 15

302

301

330

321

314

329

301

308

326

322

324

312

321

328